# EUROPÄISCHE PATENTSCHRIFT

(11) **EP 2 135 542 B1**
(45) Veröffentlichungstag und Bekanntmachung des Hinweises auf die Patenterteilung: **05.09.2012**
(21) Anmeldenummer: 09007594.6
(22) Anmeldetag: 09.06.2009
(51) Int. Cl.: A61B 1/00, A61B 17/34

(54) **Endoskop- und Schaftsystem**
Endoscope and shaft system
Système d'endoscope et d'arbre

(30) Priorität: 19.06.2008 DE 102008029301
(43) Veröffentlichungstag der Anmeldung: 23.12.2009
(73) Patentinhaber: Richard Wolf GmbH, 75438 Knittlingen (DE)
(72) Erfinder: Körner, Eberhard, Dipl.-Ing., 75438 Knittlingen (DE); Freier, Mark, 75038 Oberderdingen (DE)
(74) Vertreter: Hemmer, Arnd

(56) Entgegenhaltungen:
- EP-A- 0 455 188
- DE-A1- 4 444 049
- DE-A1- 19 935 725
- DE-A1-102007 032 201
- DE-U1-202004 014 828

## Beschreibung

Die Erfindung betrifft ein Endoskop- und Schaftsystem zur Verwendung bei der endoskopischen Wirbelsäulenchirurgie, insbesondere im Bereich der Halswirbelsäule.

Bei der endoskopischen Wirbelsdulenchirurgie, insbesondere im Bereich der Halswirbelsäule, ist anatomisch bedingt nur ein kleiner Zugangskanal möglich und dementsprechend können nur kleinkalibrige Endoskop- und Schaftsysteme verwendet werden. Die optimale Platzierung der Zugangsschäfte ist von vornherein schwierig und eine nachträgliche Korrektur ist in der Regel nur mit verhältnismäßig hohen Manipulationskräften möglich.

EP 0 455 188 A2 bildet den nächstkommenden Stand der Technik und offenbart ein Koagulationsinstrument zum Stillen von Blutungen in Nasenhöhlen. Dieses Instrument weist einen Zugangsschaft auf, an dessen proximalem Ende ein Kupplungsgehäuse ausgebildet ist, in welchem eine Aufnahme durch eine Feder vorgespannt so gehalten ist, dass sie um ihre Längsachse in bestimmten Schritten drehbar und um ein gewisses Maß gegen die Federvorspannung axial verschiebbar ist. In der Aufnahme ist mittels eines Spannrings eine Endoskopoptik fixiert. Diese Ausgestaltung hat den Nachteil, dass das Entfernen der Endoskopoptik zum einen nur einen begrenzten Querschnitt des Arbeitskanals freigibt und zum anderen die Positionierung des Endoskops durch Lösen des Spannrings verloren geht, sodass das Endoskop nach Wiedereinsetzen in das Instrument insbesondere in axialer Richtung neu positioniert werden muss.

Es ist Aufgabe der Erfindung ein Endoskop- und Schaftsystem bereitzustellen, welches es zum einen ermöglicht, große Manipulationskräfte auf den Zugangsschaft ohne Beschädigung und Beeinträchtigung der Bewegbarkeit des Endoskops zu übertragen, und es zum anderen ermöglicht, einen größeren Querschnitt des Zugangsschaftes zur Entnahme größerer Gewebemengen freizugeben, ohne die Positionierung des Endoskops zu beeinträchtigen.

Diese Aufgabe wird durch ein Endoskop- und Schaftsystem mit den in Anspruch 1 angegebenen Merkmalen gelöst. Bevorzugte Ausführungsformen ergeben sich aus den Unteransprüchen.

Das erfindungsgemäße Endoskop- und Schaftsystem weist einen Zugangsschaft sowie ein in den Zugangsschaft einsetzbares Endoskop auf. Der Zugangsschaft hat erfindungsgemäß an seinem proximalen Ende ein Griffteil. Dies hat den Vorteil, dass über das Griffteil Manipulationskräfte direkt auf den Zugangsschaft übertragen werden können, um diesen im Operationsgebiet zu positionieren. Das eingesetzte Endoskop bleibt dabei frei von den Manipulationskräften und ist vor Beschädigungen geschützt. Auf diese Weise können vergleichsweise große Manipulationskräfte übertragen werden, ohne dass das Endoskop hierfür besonders verstärkt ausgebildet werden müsste. Das Endoskop kann somit weiterhin sehr schlank angepasst an den zur Verfügung stehenden kleinen Zugangskanal ausgebildet werden, sodass der zur Verfügung stehende Raum optimal ausgenutzt werden kann.

Das Endoskop ist durch ein Verbindungsstück gehalten. Dazu ist das Endoskop vorzugsweise lösbar mit dem Verbindungsstück verbunden. Das Verbindungsstück wiederum ist lösbar mit dem Griffteil verbunden bzw. verbindbar. So kann das Verbindungsstück gemeinsam mit dem Endoskop von dem Griffteil und dem Zugangsschaft getrennt werden, beispielsweise um andere Instrumente durch den Zugangsschaft ins Operationsgebiet zu führen. Die Handhabung des Zugangsschaftes kann dabei allein über das direkt mit dem Zugangsschaft verbundene Griffteil erfolgen. Ferner ist das Verbindungsstück, wenn es mit dem Griffteil verbunden ist um die Längsachse des Endoskops relativ zu dem Griffteil drehbar. Auf diese Weise kann das Endoskop im Zugangsschaft um seine Längsachse gedreht werden, um einen möglichst großen Bereich des Operationsgebietes mit dem Endoskop betrachten zu können.

Das Endoskop ist nicht nur drehbar sondern auch axial beweglich. Eine solche axiale Beweglichkeit kann beispielsweise dadurch erreicht werden, dass das Verbindungsstück nicht nur relativ zu dem Griffteil drehbar sondern auch noch relativ zu dem Griffteil axial beweglich ist. Dabei wird dann das Endoskop gemeinsam mit dem Verbindungsstück axial verschoben und/oder gedreht, um das distale Ende des Endoskops so zu platzieren, dass ein gewünschter Bereich des Operationsgebietes betrachtet werden kann.

Erfindungsgemäß wird somit ein Endoskop- und Schaftsystem geschaffen, welches zum einen die Übertragung vergleichsweise großer Manipulationskräfte ermöglicht und zum anderen gleichzeitig eine, vorzugsweise freie, Bewegbarkeit des Endoskops in dem Zugangsschaft ermöglicht.

Bevorzugt sind das Griffteil und/oder das Verbindungsstück derart ausgebildet, dass das in dem Verbindungsstück gehaltene Endoskop mit seiner Längsachse parallel versetzt zu der Längsachse des Zugangsschaftes angeordnet ist. Auf diese Weise wird neben dem Endoskop im Querschnitt des Zugangsschaftes ein freies Lumen geschaffen, durch welches neben dem Endoskop ein Arbeitsinstrument durch den Zugangsschaft zum Operationsgebiet geführt werden kann.

Weiter bevorzugt weist der Zugangsschaft eine Querschnittsform auf, welche in einer ersten Richtung eine größere Weite aufweist als in einer zweiten Richtung, welche normal zu der ersten Richtung ist. Das heißt der Zugangsschaft ist im Querschnitt nicht kreisförmig sondern im Wesentlichen oval ausgebildet. Dies ermöglicht es, den Zugangsschaft im Querschnitt mit möglichst geringer Höhe auszubilden, sodass er gut in den Bereich der Wirbelsäule insbesondere im Zwischenwirbelraum zuführbar ist. Gleichzeitig wird durch die im Querschnitt größere Breite ein ausreichend großes Lumen im Inneren des Zugangsschaftes geschaffen, durch welches ein Endoskop und ggf. weitere Instrumente zugeführt werden können. Wie oben beschrieben, ist bevorzugt das Endoskop so platziert, dass seine Längs- bzw. Mittelachse gegenüber der Längs- bzw. Mittelachse des Zugangsschaftes parallel versetzt ist. Das heißt idealerweise liegt im ovalen Querschnitt des Zugangsschaftes das Endoskop in der einen Hälfte und die andere Hälfte bildet ein freies Lumen, durch welches beispielsweise ein Arbeitsinstrument zugeführt werden kann.

Der Zugangsschaft ist lösbar mit dem Griffteil verbunden. Dies ermöglicht es, das Instrument beim Einführen in den Zwischenwirbelraum mit anderen Instrumenten zu kombinieren und zu Reinigungs- oder Wartungszwecken zu zerlegen oder beispielsweise unterschiedliche Zugangsschäfte mit ein und demselben Griffteil zu verbinden. Weiter bevorzugt ist die Verbindung zwischen Griffteil und Zugangsschaft so ausgebildet, dass der Zugangsschaft in zwei möglichen Winkelpositionen mit dem Griffteil verbunden werden kann. Das heißt der Zugangsschaft kann um seine Längsachse um 180 Grad gedreht in einer zweiten möglichen Position mit dem Griffteil verbunden werden. Das ermöglicht bei ovaler Ausgestaltung des Zugangsschaftes und versetzter Anordnung des Endoskops, das Endoskop wahlweise in den beiden möglichen Positionen im Zugangsschaft zu positionieren. Die Verbindung zwischen Griffteil und Zugangsschaft ist beispielsweise als lösbare Kugelrastverbindung ausgebildet.

Weiter bevorzugt weist das Griffteil an seinem distalen Ende eine Aufnahme für das proximale Ende des Zugangsschaftes auf und weist an seinem proximalen Ende eine Aufnahme für das Verbindungsstück auf, wobei diese beiden Aufnahmen zueinander in einer Richtung quer zur Längsachse des Zugangsschaftes parallel versetzt angeordnet sind. Auf diese Weise sorgt das Griffteil für die versetzte Anordnung von Endoskop und Zugangsschaft. Wenn das Endoskop mit dem Verbindungsstück verbunden ist, wird das Verbindungsstück in Richtung quer zur Längsachse des Zugangsschaftes durch das Griffteil definiert positioniert. Gleichzeitig bleibt jedoch die Drehbarkeit des Endoskops um seine Längsachse und vorzugsweise auch eine axiale Beweglichkeit des Endoskops in dem Zugangsschaft gewährleistet.

Das Griffteil und das Verbindungsstück sind in axialer Richtung lösbar zusammengesteckt. Dies ermöglicht es, dass das Verbindungsstück leicht von dem Griffteil entfernt werden kann, um beispielsweise das Endoskop, welches mit dem Verbindungsstück verbunden ist, aus dem Zugangsschaft zu entnehmen. Dies kann erforderlich sein, wenn der gesamte Querschnitt des Zugangsschaftes benötigt wird, um größere Gewebemengen aus dem Operationsgebiet zu entfernen. Dadurch, dass das Endoskop mit dem Verbindungsstück verbunden bleibt, ist es möglich, dass durch die Entnahme des Endoskops die Positionierung des Endoskops nicht verloren geht. Das heißt wenn das Verbindungsstück wieder mit dem Griffteil zusammen gesteckt wird, befindet sich das Endoskop wieder in derselben Positionierung wie vor der Entnahme. Dies wird dadurch gewährleistet, dass das Endoskop über das Verbindungsstück positioniert wird, d. h. das Verbindungsstück ein Positioniermittel zum Positionieren des Endoskops in dem Zugangsschaft aufweist. Dies kann eine Positionierung in axialer Richtung und/oder eine Positionierung bezüglich der Winkellage des Endoskops um seine Längsachse beinhalten.

Das Griffteil weist vorzugsweise am proximalen Ende eine Hülse auf, welche vom distalen Ende her in das Innere des Verbindungsstücks eingreift. Das heißt das Verbindungsstück weist an seinem distalen Ende eine Öffnung auf, welche in Größe und Querschnittsform an Form und Größe des Außenumfanges der Hülse an dem Griffteil angepasst ist. Vorzugsweise ist die Hülse kreiszylindrisch ausgebildet und die Öffnung an dem Verbindungsstück ist entsprechend zylindrisch ausgebildet. So kann eine relativ große Anlagefläche zwischen Verbindungsstück und Griffteil geschaffen werden, um eine sichere Positionierung und Führung des Verbindungsstückes an dem Griffteil sicherzustellen. Die Drehbarkeit des Endoskops um seine Längsachse wird vorzugsweise dadurch erreicht, dass die Hülse des Griffteiles sich im Inneren der distalseitigen Öffnung des Verbindungsstückes drehen kann. Das heißt, dass Verbindungsstück dreht sich, vorzugsweise frei, auf der Hülse an dem Griffteil.

Weiter ist es bevorzugt, dass das Verbindungsstück, an seinem proximalen Ende eine Befestigungsaufnahme zum lösbaren Befestigen des Endoskops aufweist. Durch diese Befestigungsaufnahme wird das Endoskop definiert an dem Verbindungsstück gehalten bzw. positioniert. Dabei ist es bevorzugt, dass das Endoskop zu Reinigungs- und Wartungszwecken von dem Verbindungsstück gelöst werden kann. Ferner ermöglichst dies, dass unterschiedliche Endoskope an ein und demselben Verbindungsstück angebracht werden können.

Die lösbare Befestigung des Endoskops ist vorzugsweise dadurch möglich, dass das Endoskop durch einen drehbaren Klemmring mit sich über den Umfang änderndem Innendurchmesser an zumindest einer Anlagefläche der Befestigungsaufnahme lösbar fixierbar ist. Der Klemmring ist so ausgebildet, dass er in einer gelösten Position so positioniert ist, dass ein größerer Abstand zwischen Klemmring und Anlagefläche gegeben ist, sodass das Endoskop zwischen beiden bewegbar ist. Durch Verdrehen des Klemmringes wird ein Bereich mit kleinerem Innendurchmesser mit der Anlagefläche zur Überdeckung gebracht. Dadurch verkleinert sich der radiale Abstand zwischen Klemmring und Anlagefläche, sodass das Endoskop mit einem korrespondierenden Befestigungsabschnitt zwischen dem Klemmring und der Anlagefläche eingeklemmt werden kann. Dabei ist die Befestigungsaufnahme weiter bevorzugt so ausgestaltet, dass neben dieser kraftschlüssigen Klemmung auch eine formschlüssige Positionierung des Endoskops gegeben ist, um das Endoskop in einer definierten Lage zu halten.

Die Befestigungsaufnahme ist vorzugsweise von einer Hülse gebildet, welche am proximalen Ende einen sektorförmigen Ausschnitt aufweist, wobei die dem Ausschnitt zugewandten Stirnflächen der Hülse Anlageflächen für das Endoskop bilden. Das Endoskop weist einen korrespondierenden Befestigungsabschnitt auf, welcher in diesen sektorförmigen Ausschnitt eingesetzt werden kann. Dabei kommen Anlageflächen, welche an dem Endoskop ausgebildet sind, an den Anlageflächen der Ausnehmung, d. h. den Stirnflächen an dem Ausschnitt der Hülse zur Anlage. Wenn ein Klemmring, wie er vorangehend beschrieben wurde vorgesehen ist, wird durch Verdrehen des Klemmringes vorzugsweise das Endoskop mit seinen Anlageflächen gegen diese Stirnflächen der Hülse gedrückt und so an der Hülse fixiert.

Gemäß einer weiteren bevorzugten Ausführungsform weist das Verbindungsstück eine Verstelleinrichtung auf, mittels welcher das Endoskop relativ zu dem Griffteil axial bewegbar ist. Das heißt über das Verbindungsstück ist eine axiale Positionierung des Endoskops in dem Zugangsschaft möglich. Die Anordnung der Verstelleinrichtung an dem Verbindungsstück hat den Vorteil, dass, wenn das Verbindungsstück von dem Griffteil getrennt wird, die Einstellung der Verstelleinrichtung hiervon unabhängig ist bzw. nicht beeinflusst wird, sodass dann wenn das Verbindungsstück wieder mit dem Griffteil verbunden wird, die vorherige Positionierung des Endoskops in dem Zugangsschaft wiederhergestellt ist.

Zur Ausbildung der Verstelleinrichtung weist das Verbindungsstück weiter bevorzugt zwei ineinander greifende Hülsen auf, welche über ein Gewinde miteinander in Eingriff sind, wobei an einer ersten Hülseein Stellring zum Verdrehen angeordnet ist und damit axialen Verstellen der ersten Hülse relativ zu der zweiten Hülse. An einer dieser Hülsen ist bevorzugt das Endoskop in der oben beschrieben Weise befestigt. Der Stellring ist bevorzugt an der anderen Hülse angebracht. An der Hülse ohne Stellring ist vorzugsweise ein Verschlussring vorgesehen, um damit das Endoskop festzulegen. Die Hülse mit dem Stellring ist weiter bevorzugt auf eine am proximalen Ende des Griffteils ausgebildete Hülse aufgesteckt, wie es oben beschrieben wurde. So kann die Hülse mit dem Stellring auf der Hülse des Griffteiles verdreht werden, um das Endoskop um seine Längsachse zu drehen. Die Hülse an dem Griffteil erstreckt sich weiter bevorzugt soweit in das Innere des Verbindungsstückes hinein, dass es auch in die zweite Hülse, welche mit der Hülse mit dem Stellring über das Gewinde im Eingriff ist, eingreift. Die äußere der beiden Gewindehülsen weist weiter bevorzugt an ihrem dem Gewinde abgewandten Axialende einen sich radial nach innen erstreckenden Kragen auf, welcher einen Innendurchmesser im Wesentlichen wie die innere der beiden Gewindehülsen aufweist. Dadurch wird sichergestellt, dass beide Gewindehülsen am Außenumfang der Hülse des Griffteiles zur Anlage kommen können. Dies stellt eine verbesserte Führung und Positionierung des Verbindungsstückes an dem Griffteil sicher.

Weiter ist es bevorzugt, dass zwischen den beiden Hülsen, welche über die Gewinde miteinander in Eingriff sind, ein Federelement angeordnet ist, welches eine Klemmkraft zwischen beiden Hülsen bewirkt. Ein solches Federelement kann dadurch das Spiel in dem Gewinde ausschalten oder konstant halten, sodass die beiden Hülsen vorzugsweise spielfrei mit einer gewissen Schwergängigkeit zueinander verstellt werden können. Auf diese Weise wird eine sehr präzise Verstellung möglich und eine unbeabsichtigte Verstellung verhindert. Das Federelement kann als Federzunge ausgebildet sein, welche sich in Umfangsrichtung erstreckt und durch eine im Wesentlichen U-förmig verlaufende Trennfuge in der Hülsenwandung ausgebildet ist. Auf diese Weise kann das Federelement beispielsweise in der äußeren der beiden Hülsen ausgebildet sein und von einem umgebenden ringförmigen Klemmelement radial nach innen gegen die innere der Gewindehülsen gedrückt werden.

Weiterhin ist es bevorzugt, dass das Griffteil und das Verbindungsstück im Inneren ein sich axial erstreckendes freies Lumen aufweisen, welches mit dem freien Lumen im Inneren des Zugangsschaftes fluchtet. Dies bedeutet, dass, wenn das Endoskop in den Zugangsschaft eingesetzt ist, dort neben dem Endoskop ein freies Lumen verbleibt, welches proximalseitig mit dem freien Lumen im Griffteil und dem Verbindungsstück fluchtet, sodass sich ein durchgehender freier Kanal vom proximalen zum distalen Ende des Instrumentes erstreckt. Durch diesen können Instrumente eingeführt werden. Um dieses durchgehende freie Lumen zu schaffen, ist das Endoskop in dem Verbindungsstück und in dem Griffteil vorzugsweise außermittig am Innenumfang angeordnet, sodass daneben das freie Lumen verbleibt.

Weiter bevorzugt ist das Endoskop im Bereich des Verbindungsstückes im Querschnitt dachkantförmig ausgebildet, sodass es einen Innenquerschnitt des Verbindungsstückes im Wesentlichen nur in einem Sektor kleiner 180 Grad ausfüllt. Durch diese Ausgestaltung wird erreicht, dass auch beim Verdrehen des Endoskops gemeinsam mit dem Verbindungsstück das durchgehende freie Lumen über einen möglichst großen Verdrehwinkel erhalten bleibt. In dem Verbindungsstück verbleibt bei eingesetztem Endoskop somit ein freier Sektor welcher größer 180 Grad ist, sodass ein etwa bogenförmiger Freiraum verbleibt. Beim Verdrehen des Verbindungsstückes überstreicht dieser freie Sektor bzw. Bogen das proximale Ende des freien Lumens des Zugangsschaftes, sodass ein durchgehender Durchgang auch durch das Verbindungsstück hindurch beim Verdrehen erhalten bleibt.

Nachfolgend wir die Erfindung beispielhaft anhand der beigefügten Figuren beschrieben. In diesen zeigt:

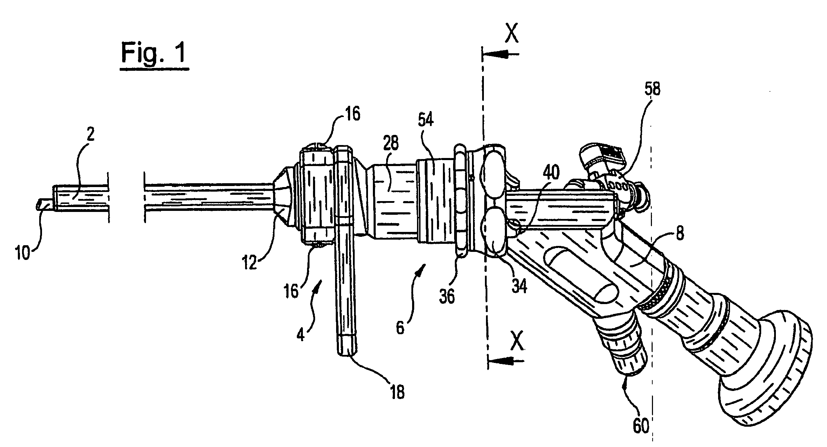

- Fig. 1: eine schematische Gesamtansicht eines erfindungsgemäßen Endoskop- und Schaftsystems im zusammengesetzten Zustand,

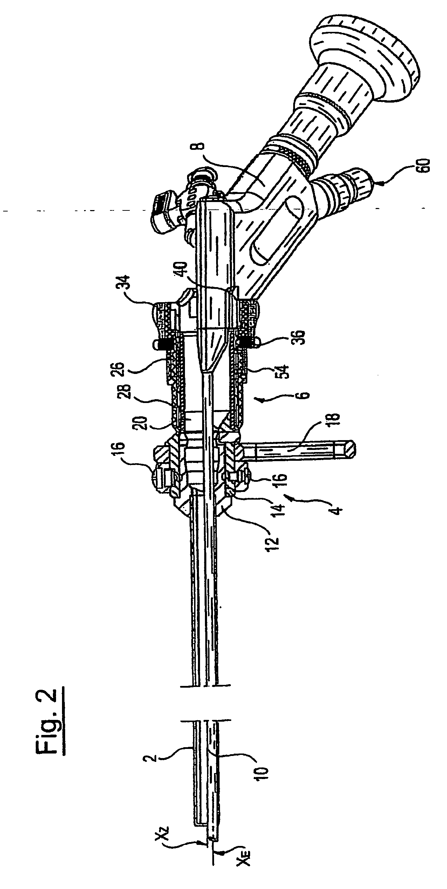

- Fig. 2: eine teilweise geschnittene Ansicht des Endoskop- und Schaftsystems gemäß Fig. 1,

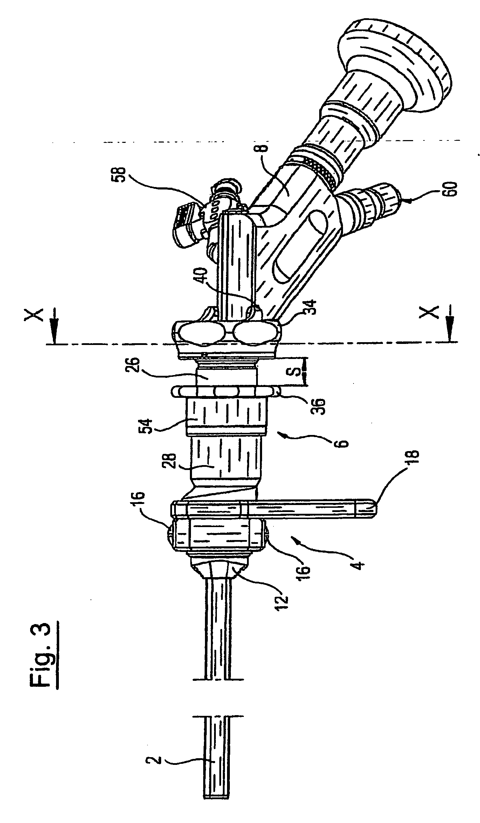

- Fig. 3: ein Endoskop- und Schaftsystem gemäß Fig. 1 mit proximalwärts verlagertem Endoskop,

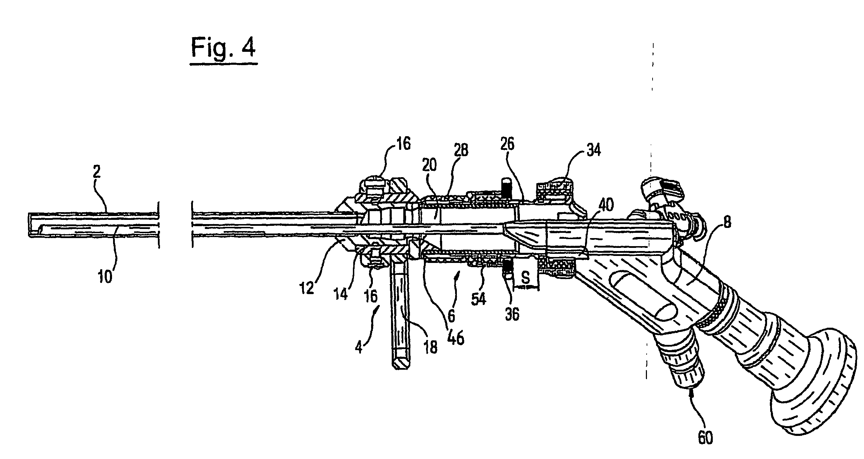

- Fig. 4: eine Schnittansicht des in Fig. 3 gezeigten Endoskop- und Schaftsystems,
- Fig. 5: eine Querschnittansicht des Zugangsschaftes mit darin liegendem Endoskop,
- Fig. 6: eine Draufsicht auf das Instrument gemäß Fig. 1 bis 4 von der proximalen Seite her gesehen,
- Fig. 7: eine Gesamtansicht des Instrumentes gemäß Fig. 1 bis 6 mit einem eingesetzten Hilfsinstrument,
- Fig. 8: eine Detailansicht des Griffteiles,
- Fig. 9: eine Schnittansicht des Verbindungsstückes,
- Fig. 10: eine Schnittansicht entlang der Linie X-X in Fig. 1,
- Fig. 11: eine Ansicht gemäß Fig. 10, wobei sich das Endoskop im geklemmten Zustand befindet,
- Fig. 12: eine Detailansicht der äußeren Hülse des Verbindungsstückes und
- Fig. 13: eine Schnittansicht entlang der Linie XIII-XIII in Fig. 12.

Das als Ausführungsbeispiel gezeigte erfindungsgemäße Endoskop- und Schaftsystem besteht im Wesentlichen aus zwei Baugruppen. Die erste Baugruppe wird gebildet von einem Zugangsschaft 2 und einem Griffteil 4, welches mit dem Zugangsschaft 2 lösbar verbunden ist. Dazu ist das Griffteil 4 an seinem distalen Ende bekannterweise mit einer Aufnahme für das proximale Ende des Zugangsschaftes 2 mittels einer RastVerbindung ausgebildet.

Die zweite Baugruppe ist gebildet aus einem Verbindungsstück 6 und einem Endoskop 8. Das Endoskop 8 ist vom proximalen Ende her in das Verbindungsstück 6 eingesetzt, sodass sich der Optikschaft 10 des Endoskops distalwärts in den Zugangsschaft 2 hinein erstreckt. Das Endoskop 8 ist lösbar mit dem Verbindungsstück 6 verbunden, wie weiter unten näher beschrieben wird.

Der Zugangsschaft 2 endet an seinem proximalen Ende an einem Anschlussstück 12. Dieses ist in eine distalseitige Aufnahme 14 des Griffteils 4 eingesetzt und dort über die Rastelemente 16 lösbar formschlüssig mit dem Griffteil 4 verbunden.

Wesentlicher Bestandteil des Griffteiles 4 ist der Griff 18, welcher sich radial nach außen erstreckt. Über die Aufnahme 14 und das Anschlussstück 12 ist der Griff direkt mit dem Zugangsschaft 2 verbunden, sodass über den Griff 18 Manipulationskräfte direkt auf den Zugangsschaft 2 übertragen werden können, ohne dass das Endoskop 8 mit diesen Kräften belastet wird. Auf diese Weise können auf den Zugangsschaft 2 vergleichsweise hohe Manipulationskräfte aufgebracht werden.

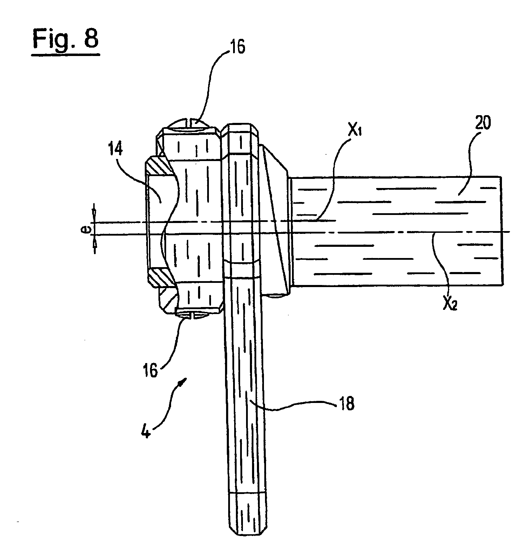

Wie am besten in der Detailansicht in Fig. 8 zu sehen ist, weist das Griffteil 4 am proximalen Ende eine zylindrische Hülse 20 auf. Diese dient zur Verbindung mit dem Verbindungsstück 6 und wird zu diesem Zweck vom distalen Ende her in das Verbindungsstück 6 eingesteckt. Das Griffteil 4 und das Verbindungsstück 6 werden somit einfach zusammengesteckt und können leicht voneinander getrennt werden. Wie in Fig. 8 zu erkennen ist, sind die Längsachse X₁ der zylindrischen Aufnahme 14 und die Längsachse X₂ der Hülse 20 um ein Maß e parallel versetzt zueinander angeordnet, d. h. in lateraler Richtung versetzt.

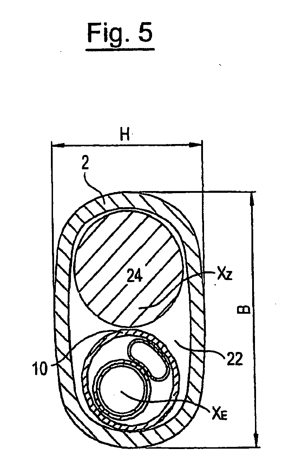

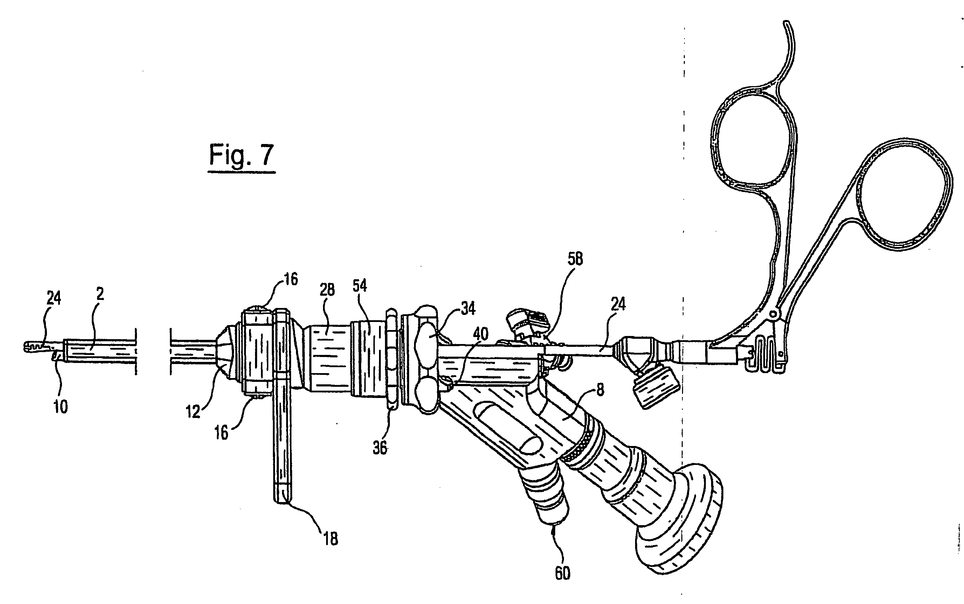

Der Versatz e der Hülse 20 gegenüber der Aufnahme 14 in dem Griffteil 4 steht im Zusammenhang mit der Ausgestaltung des Zugangsschaftes 2. In Fig. 5 ist der Zugangsschaft im Querschnitt gezeigt. Es ist zu erkennen, dass der Zugangsschaft 2 einen im Wesentlichen ovalen Querschnitt aufweist. Das heißt die Erstreckung bzw. Breite B des Zugangsschaftes in einer ersten Querschnittsrichtung ist größer als die Erstreckung bzw. Höhe H in einer zweiten zu der ersten Querschnittsrichtung normalen Querschnittsrichtung. Im Inneren des Zugangsschaftes 2 ist der Optikschaft 10 nicht zentral sondern seitlich versetzt in einer Hälfte des Innenquerschnittes des Zugangsschaftes 2 angeordnet. Das heißt die Längsachse X_{E} des Optikschaftes 10 ist parallel versetzt zu der Längs- bzw. Mittelachse Xz des Zugangsschaftes 2, welche sich in Verlängerung der Längsachse X₁ der Aufnahme 14 erstreckt. Auf diese Weise verbleibt ein freies Lumen 22 in welches ein Arbeitsinstrument 24 eingesetzt werden kann. Dies kann beispielhaft eine Zange sein, wie Sie in Fig. 7 gezeigt ist. So füllen der Endoskop- bzw. Optikschaft 10 und der Arbeitseinsatz 24 den Innenquerschnitt des Zugangsschaftes 2 optimal aus, wobei gleichzeitig die Höhe H des Zugangsschaftes 2 gering gehalten wird, sodass das Instrument auch über enge Zugangskanäle zum Operationsgebiet bewegt werden kann. Die Anordnung des Optikschaftes 10 im Inneren des Zugangsschaftes 2 in einer Hälfte seitlich versetzt bezüglich der Mittelachse Xz des Zugangsschaftes 2 wird durch den Versatz e zwischen Hülse 20 und Aufnahme 14 in dem Griffteil 4 erreicht. Der Versatz e stellt dabei gleichzeitig sicher, dass das Endoskop 8 um die Längsachse des Endoskop- bzw. Optikschafts 10 drehbar bleibt, um das Blickfeld im Operationsgebiet drehen zu können. Diese Drehung wird dadurch erreicht, dass das Verbindungsstück 6 auf der Hülse 20 des Griffteiles 4 um die Längsachse X₂ der Hülse 20 verdreht werden kann. Das Endoskop 8 ist dabei in dem Verbindungsstück 6 so positioniert, dass sich die Längsachse X_{E} des Optikschafts 10 entlang der Längsachse X₂ des Verbindungsstückes 6 und der Hülse 20 erstreckt.

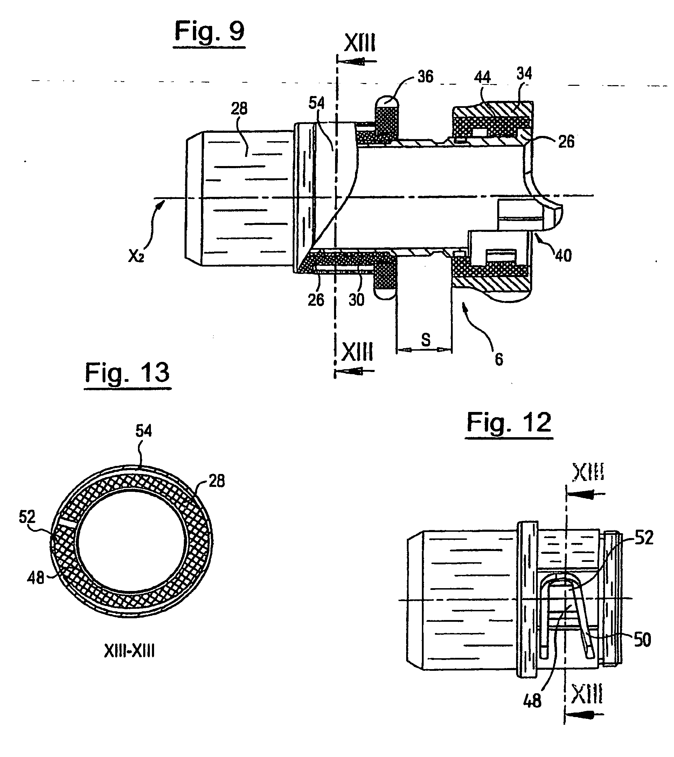

Das Verbindungsstück 6 besteht, wie am besten in der Detailansicht von Figur 9 zu erkennen ist, im Wesentlichen aus einer inneren Hülse 26 und einer äußeren Hülse 28, welche über Gewinde 30 und 32 miteinander in Eingriff sind. Das Gewinde 30 ist als Außengewinde auf der inneren Hülse 26 ausgebildet, das Gewinde 32 ist als Innengewinde auf der Innenseite der äußeren Hülse 28 ausgebildet. Die innere Hülse 26 erstreckt sich proximalwärts aus der äußeren Hülse 28 hinaus. Dort ist sie mit einem Verschlussring 34 am Außenumfang versehen. Die äußere Hülse 28 ist am Außenumfang mit einem Stellring 36 versehen, welcher radial nach außen auskragt. Über dem Verschlussring 34 und den Stellring 36 können die innere Hülse 26 und die äußere Hülse 28 ergriffen und gegeneinander verdreht werden. Durch den Gewindeeingriff bewegen sie sich dabei in axialer Richtung auseinander oder zusammen, je nach Drehrichtung. Auf diese Weise wird eine axiale Zustellung um das Maß s möglich. Am proximalen Ende der inneren Hülse 26 ist das Endoskop 8 festgelegt, wie nachfolgend näher erläutert werden wird. Dadurch wird bei Verstellung der äußeren Hülse 28 gegenüber der inneren Hülse 26 auch das Endoskop relativ zu dem über die äußere Hülse 28 mit dem Verbindungsstück 6 verbundenen Griffteil 4 und damit in axialer Richtung in dem Zugangsschaft 2 bewegt. Auf diese Weise kann das distale Ende des Endoskops ebenfalls um das Maß s durch Verdrehen der beiden Hülsen des Verbindungsstückes 6 bewegt werden.

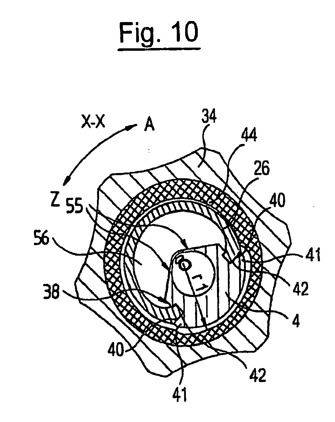

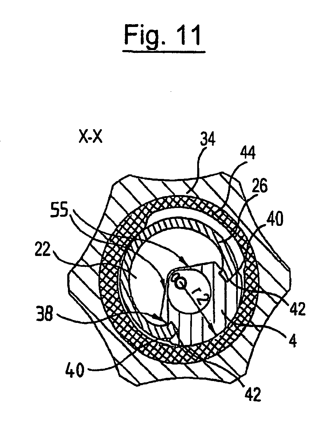

Zur Verbindung mit dem Endoskop 8 weist die innere Hülse 26 an ihrem proximalen Ende einen zum proximalen Ende hin geöffneten sektorförmigen Ausschnitt 38 auf. Die sich in Längsrichtung der Hülse 26 erstreckenden und die Ausschnitt 38 begrenzenden Stirnflächen der Hülse 26 bilden zwei Anlageflächen 40. Das Endoskop 8 weist zwei zu entgegengesetzten Seiten auskragende Schultern 41 auf, welche sich parallel zur Längsrichtung X_{E} des Endoskops erstrecken. Diese Schultern 41 bilden Anlageflächen 42, welche den Anlageflächen 40 an der Hülse 26 gegenüberliegen und mit diesen zur Anlage kommen. Die Breite des Endoskops 8 zwischen den Schultern 41 ist so gewählt, dass sie der Breite des Ausschnitts 38 entspricht. So kann das Endoskop 8 formschlüssig in den Ausschnitt 38 in der Hülse eingesetzt werden. Dabei ist das Endoskop 8 so gelegen, dass der Endoskopschaft 10 sich zentral in der Hülse 26 in Richtung deren Längsachse X₂ erstreckt. Fixiert wird das Endoskop über einen Klemmring 44, welcher die innere Hülse 26 am Außenumfang umgibt und im Inneren des ringförmigen Verschlussringes 34 gelegen ist. Der Klemmring 44 ist mit dem Verschlussring 34 um die Hülse 26 in Richtung der Pfeile A und Z drehbar. Die Drehung in Richtung A bewirkt eine Drehung in die geöffnete Position, welche in Fig. 10 gezeigt ist. Die Drehung in Richtung Z bewirkt eine Drehung in die geschlossene Position, welche in Figur 11 dargestellt ist. In der geschlossenen Position wird das Endoskop 8 gegen die Anlageflächen 40 gedrückt und so geklemmt. Dies erfolgt dadurch, dass der Klemmring 44 am Innenumfang einen sich über den Umfang ändernden Durchmesser bzw. Radius aufweist. In der gelösten Stellung in Fig. 10 ist ein Bereich des Klemmringes über dem Endoskop 8 gelegen, welcher einen größeren Radius r₁ aufweist. So verbleibt ein gewisses Spiel zwischen Klemmring und Endoskop 8, sodass dieses nicht geklemmt ist. Bei Drehung in Richtung des Pfeils Z kommt der Bereich des Klemmringes 44 mit geringerem Innenradius r₂ über dem Endoskop 8 zu liegen, wie in Fig. 11 gezeigt, sodass das Endoskop 8 mit seinen Anlageflächen 42 spielfrei gegen die Anlageflächen 40 an der inneren Hülse 26 gedrückt wird und somit an der Hülse 26 und damit in dem Verbindungsstück 6 fixiert wird.

Wenn das Endoskop 8 in dem Verbindungsstück 6 fixiert ist, kann es gemeinsam mit dem Verbindungsstück 6 von dem Griffteil 4 abgenommen und somit aus dem Zugangsschaft 2 entnommen werden. Dies kann erforderlich sein, wenn der gesamte Innenquerschnitt des Zugangsschaftes benötigt wird, um beispielsweise größere Gewebemengen aus dem Operationsgebiet zu entfernen. Da bei dieser Entnahme des Endoskops 8 aus dem Zugangsschaft 2 die Hülsen 26 und 28 des Verbindungsstückes 6 nicht gegeneinander verdreht werden, bleibt die Positionierung des Endoskops 8 in axialer Richtung erhalten. Das heißt, wenn das Endoskop 8 wieder in den Zugangsschaft 2 eingesetzt und das Verbindungsstück 6 mit dem Griffteil 4 verbunden ist, befindet sich das Endoskop 8 wieder in derselben axialen Position wie vor der Entnahme.

Der Eingriff von Verbindungsstück 6 und Griffteil 4 erfolgt in der Weise, dass sich die proximalseitige Hülse 20 des Griffteiles 4 in die innere Hülse 26 des Verbindungsstückes 6 hinein erstreckt. Der Außendurchmesser der Hülse 20 entspricht dabei dem Innendurchmesser der inneren Hülse 26. Zusätzlich ist die äußere Hülse 28 an ihrem distalen Ende mit einem radial nach innen gerichteten ringförmigen Kragen 46 versehen, welcher ebenfalls am Außenumfang der Hülse 20 zur Anlage kommt. So ist auch dann, wenn die innere Hülse in proximaler Richtung verstellt ist, und der maximale Abstand s zwischen inneren Hülse 26 und der äußeren Hülse 28 erreicht wird, eine sichere Anlage des Verbindungsstückes 6 auf der Hülse 20 gewährleistet.

Um eine spielfreie und gewisse schwergängige Verstellung der Hülsen 26 und 28 über den Eingriff der Gewinde 30, 32 zu gewährleisten, ist die äußere Hülse 28 im Bereich des Gewindeeingriffes mit der inneren Hülse 26 mit einem Federelement versehen. Das Federelement ist als eine sich in Umfangsrichtung erstreckende Zunge 48 ausgebildet. Die Zunge 48 wird dabei durch eine im Wesentlichen U-förmig verlaufende Trennfuge 50 in der Wandung der Hülse 28 gebildet. Diese Trennfuge 50 erstreckt sich durch die gesamte Stärke der Wandung der Hülse 28 vom Außenumfang zum Innenumfang. Die so gebildete Zunge 48 ist an ihrem freien Ende mit einem radial nach außen vorstehenden Wulst 52 versehen. Wie in Fig. 13 zu erkennen ist, ist der Außenumfang der Hülse 28 in dem Bereich, in dem die Zunge 48 ausgebildet ist, im Wesentlichen oval. In diesem Bereich ist die Hülse umfänglich von einem Spannring 54 umgeben. Der Wulst 52 liegt dabei am Innenumfang des Spannringes 54 an. Dies bewirkt, dass die Zunge 48 nach innen gegen den Außenumfang der inneren Hülse 26 gedrückt wird. Auf diese Weise kommt es zu einer Klemmung zwischen der inneren Hülse 26 und der äußeren Hülse 28, welche das Spiel aus dem Eingriff der Gewinde 30 und 32 eliminiert.

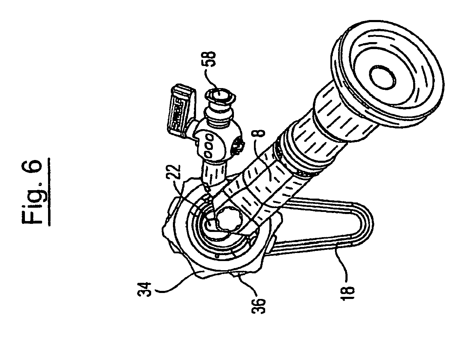

Wie in den Fig. 6, 10 und 11 zu erkennen ist, ist das Endoskop 8 in dem Bereich, mit welchem es in die Ausnehmung bzw. dem Ausschnitt 38 der Hülse 26 eingreift im Querschnitt dachkantförmig ausgebildet, wobei die schrägen Außenseiten 55 im Wesentlichen tangential zum Außenumfang des Optikschafts 10 verlaufen. Diese Ausgestaltung des Endoskops in diesem Bereich hat den Vorteil, dass das Endoskop im Querschnitt vom Innenraum der Hülse 26 nur einen Sektor kleiner 180 Grad überdeckt. Der größte Teil des Querschnittes bleibt frei und bildet ein freies Lumen 56. Dieses überdeckt die Stirnseite des Zugangsschaftes 2 im Bereich dessen freien Lumens 22, welches zur Aufnahme des Arbeitsinstrumentes 24 vorgesehen ist. Auf diese Weise kann auch dann, wenn ein solches Arbeitsinstrument 24 in dem Zugangsschaft 2 eingesetzt ist, dennoch das Endoskop 8 noch über einen großen Winkelbereich um seine Längsachse X_{E} verdreht werden, indem die Hülse 26 auf der Hülse 20 verdreht wird. Das freie Lumen 56 der Hülse 26 überstreicht dabei bogenförmig den Bereich, welcher zur Aufnahme des Arbeitsinstrumentes 24 vorgesehen ist. So liegen das freie Lumen 56 der Hülse 26 und das freie Lumen 22 des Zugangsschaftes in einer Flucht und bilden ein gemeinsames freies Lumen vom proximalen zum distalen Ende. So kann das Endoskop 8 soweit gedreht werden, bis das Arbeitsinstrument 24 an eine der beiden schrägen Flächen 55 des Dachkantprofils des Endoskops 8 anstößt.

Insgesamt wird somit ein Endoskop- und Schaftsystem bereitgestellt, welches zum einen ermöglicht, große Manipulationskräfte auf den Zugangsschaft 2 zu übertragen und gleichzeitig eine große Beweglichkeit des Endoskops 8 im Inneren des Zugangsschaftes 2 ermöglicht. Das Endoskop 8 kann in axialer Richtung X_{E} bewegt werden und um seine Längsachse X_{E} verdreht werden. Gleichzeitig bleibt das Endoskop 8 völlig frei von Manipulationskräften, welche von dem Griff 18 auf den Zugangsschaft übertragen werden. Das Endoskop 8 ist im Übrigen in herkömmlicher Weise ausgebildet, d. h. es weist eine Optik, Lichtleiter zur Beleuchtung und einen Spülkanal auf. Für diese sind proximalseitig ein Spülanschluss 58 und ein Lichtleiteranschluss 60 vorgesehen.

Anstelle des optischen Systems kann ein CCD-Element oder dergleichen und anstelle der Lichtleiter können ein oder mehrere LED, wie beispielsweise bei einem Videoendoskop üblich, vorgesehen sein.

### Bezugszeichenliste

- 2 -: Zugangsschaft
- 4 -: Griffteil
- 6 -: Verbindungsstück
- 8 -: Endoskop
- 10 -: Optikschaft
- 12 -: Anschlussstück
- 14 -: Aufnahme
- 16 -: Rastelement
- 18 -: Griff
- 20 -: Hülse
- 22 -: freies Lumen
- 24 -: Arbeitsinstrument
- 26 -: innere Hülse
- 28 -: äußere Hülse
- 30, 31 -: Gewinde
- 34 -: Verschlussring
- 36 -: Stellring
- 38 -: Ausschnitt
- 40 -: Anlageflächen
- 41 -: Schultern
- 42 -: Anlageflächen
- 44 -: Klemmring
- 46 -: Kragen
- 48 -: Zungen
- 50 -: Spalt
- 52 -: Wulst
- 54 -: Spannring
- 55 -: Flächen
- 56 -: Lumen
- 58 -: Spülanschluss
- 60 -: Lichtleiteranschluss
- Z, A -: Drehrichtungen des Klemmringes 44
- s -: axialer Verstellweg
- e -: Exzentrizität
- r₁, r₂ -: Radien Klemmring
- Xz -: Längsachse des Zugangsschaftes
- X_{E} -: Längsachse des Endoskopschaftes
- X₁ -: Längsachse der Aufnahme 14
- X₂ -: Längsachse der Hülse 20 und des Verbindungsstücks 6

## Patentansprüche

1. Endoskop- und Schaftsystem mit einem Zugangsschaft (2) und einem
in den Zugangsschaft (2) einsetzbaren Endoskop (8), wobei der Zugangsschaft (2) an seinem proximalen Ende ein Griffteil (4) aufweist und das Endoskop (8) in einem Verbindungsstück (6) gehalten ist, **dadurch gekennzeichnet, dass**
das Griffteil (4) und das Verbindungsstück (6) in axialer Richtung lösbar zusammengesteckt sind, und das Verbindungsstück, wenn es mit dem Griffteil verbunden ist, um die Längsachse des Endoskops (8) relativ zu dem Griffteil (4) drehbar und axial verstellbar ist.

2. Endoskop- und Schaftsystem nach Anspruch 1, bei welchem das Griffteil (4) und/oder das Verbindungsstück (6) derart ausgebildet sind, dass das in dem Verbindungsstück (6) gehaltene Endoskop (8) mit seiner Längsachse parallel versetzt zu der Längsachse des Zugangsschaftes (2) angeordnet ist.

3. Endoskop- und Schaftsystem nach einem der vorangehenden Ansprüche , bei welchem der Zugangsschaft (2) eine Querschnittsform hat, welche in einer ersten Richtung eine größere Weite aufweist als in einer zweiten zu der ersten Richtung normalen Richtung.

4. Endoskop- und Schaftsystem nach einem der vorangehenden Ansprüche, bei welchem der Zugangsschaft (2) lösbar mit dem Griffteil (4) verbunden ist.

5. Endoskop- und Schaftsystem nach einem der vorangehenden Ansprüche, bei welchem das Griffteil (4) an seinem distalen Ende eine Aufnahme (14) für das proximale Ende des Zugangsschaftes (2) aufweist und an seinem proximalen Ende eine Aufnahme (20) für das Verbindungsstück (6) aufweist, wobei diese beiden Aufnahmen (14, 20) in einer Richtung (e) quer zur Längsachse des Zugangsschaftes (2) zueinander versetzt angeordnet sind.

6. Endoskop- und Schaftsystem nach einem der vorangehenden Ansprüche, bei welchem das Griffteil (4) am proximalen Ende eine Hülse (20) aufweist, welche vom distalen Ende her in das Innere des Verbindungsstückes (6) eingreift.

7. Endoskop- und Schaftsystem nach einem der vorangehenden Ansprüche, bei welchem das Verbindungsstück (6) an seinem proximalen Ende eine Befestigungsaufnahme (38) zum lösbaren Befestigen des Endoskops (8) aufweist.

8. Endoskop- und Schaftsystem nach Anspruch 7, bei welchem das Endoskop (8) durch einen drehbaren Klemmring (44) mit sich über den Umfang änderndem Innendurchmesser an zumindest einer Anlagefläche (40) der Befestigungsaufnahme (38) lösbar fixiert ist.

9. Endoskop- und Schaftsystem nach Anspruch 8, bei welchem die Befestigungsaufnahme von einer Hülse (26) gebildet wird, welche am proximalen Ende einen sektorförmigen Ausschnitt (38) aufweist, wobei die dem Ausschnitt (38) zugewandten Stirnflächen der Hülse (26) Anlageflächen (40) für das Endoskop (8) bilden.

10. Endoskop- und Schaftsystem nach einem der vorangehenden Ansprüche, bei welchem das Verbindungsstück (6) eine Verstelleinrichtung (30, 32) aufweist, mittels welcher das Endoskop (8) relativ zu dem Griffteil (4) axial bewegbar ist.

11. Endoskop- und Schaftsystem nach Anspruch 10, bei welchem das Verbindungsstück (6) zwei ineinander greifende Hülsen (26, 28) aufweist, welche über ein Gewinde (30, 32) miteinander in Eingriff sind, wobei an einer ersten Hülse (28) ein Stellring (36) angeordnet ist zum Verdrehen und damit axialen Verstellen der ersten Hülse (28) relativ zu der zweiten Hülse (26).

12. Endoskop- und Schaftsystem nach Anspruch 11, bei welchem zwischen den beiden Hülsen (26, 28) ein Federelement (48) angeordnet ist, welches eine Klemmkraft zwischen beiden Hülsen (26, 28) bewirkt.

13. Endoskop- und Schaftsystem nach einem der vorangehenden Ansprüche, bei welchem das Griffteil (4) und das Verbindungsstück (6) im Inneren ein sich axial erstreckendes freies Lumen (56) aufweisen, welches mit dem freien Lumen (22) im Inneren des Zugangsschaftes (2) fluchtet.

14. Endoskop- und Schaftsystem nach einem der vorangehenden Ansprüche, bei welchem das Endoskop (8) im Bereich des Verbindungsstückes (6) im Querschnitt dachkantförmig ausgebildet ist, so dass es einen Innenquerschnitt des Verbindungsstückes (6) im Wesentlichen nur in einem Sektor kleiner 180 Grad ausfüllt.

## Claims

1. An endoscope system and shank system with an access shank (2) and with an endoscope (8) insertable into the access shank (2), wherein the access shank (2) at its proximal end comprises a grip part (4), and the endoscope (8) is held in a connection piece (6),
**characterised in that**
the grip part (4) and the connection piece (6) are releasably stuck together in the axial direction, and the connection piece when it is connected to the grip part, is rotatable about the longitudinal axis of the endoscope (8) relative to the grip part (4) and is axially adjustable, relative to the grip part (4).

2. An endoscope system and shank system according to claim 1, with which the grip part (4) and/or the connection piece (6) are designed in a manner such that the endoscope (8) held in the connection piece (6) is arranged with its longitudinal axis offset parallel to the longitudinal axis of the access shank (2).

3. An endoscope system and shank system according to one of the preceding claims, with which the access shank (2) has a cross-sectional shape which in a first direction has a greater width than in a second direction normal to the first direction.

4. An endoscope system and shank system according to one of the preceding claims, with which the access shank (2) is releasably connected to the grip part (4).

5. An endoscope system and shank system according to one of the preceding claims, with which the grip part (4) at its distal end comprises a receiver (14) for the proximal end of the access shank (2) and at its proximal end comprises a receiver (20) for the connection piece (6), wherein these two receivers (14, 20) are arranged offset to one another in a direction (e) transverse to the longitudinal axis of the access shank (2).

6. An endoscope system and shank system according to one of the preceding claims, with which the grip part (4) at the proximal end comprises a sleeve (20) which engages from the distal end into the inside of the connection piece (6).

7. An endoscope system and shank system according to one of the preceding claims, with which the connection piece (6) at its proximal end comprises a fastening receiver (38) for the releasable fastening of the endoscope (8).

8. An endoscope system and shank system according to claim 7, with which the endoscope (8) is releasably fixed on at least one bearing-contact surface (40) of the fastening receiver (38) by way of a rotatable clamping ring (44) with an inner diameter changing over the periphery.

9. An endoscope system and shank system according to claim 8, with which the fastening receiver is formed by a sleeve (26) which at the proximal end comprises a sector-like recess (38), wherein the end-faces of the sleeve (26) which face the recess (38) form bearing-contact surfaces (40) for the endoscope (8).

10. An endoscope system and shank system according to one of the preceding claims, with which the connection piece (6) comprises an adjustment device (30, 32), by way of which the endoscope (8) is axially movable relative to the grip part (4).

11. An endoscope system and shank system according to claim 10, with which the connection piece (6) comprises two sleeves (26, 28) which engage into one another and which are engaged with one another via a thread (30, 32), wherein a setting ring (36) for rotating and thus axially adjusting a first sleeve (28) relative to the second sleeve (26) is arranged on the first sleeve (28).

12. An endoscope system and shank system according to claim 11, with which a spring element (48) which effects a clamping force between both sleeves (26, 28) is arranged between the two sleeves (26, 28)

13. An endoscope system and shank system according to one of the preceding claims, with which the grip part (4) and the connection piece (6) in the inside have an axially extending free lumen (56) which is aligned to the free lumen (22) in the inside of the access shank (2).

14. An endoscope system and shank system according to one of the preceding claims, with which the endoscope (8) in cross section is designed in a roof-prism-shaped manner in the region of the connection piece, so that it fills out an inner cross section of the connection piece (6) essentially only in a sector smaller than 180 degrees.

## Revendications

1. Système endoscope et tige, comprenant une tige d'accès (2) et un endoscope (8) pouvant être inséré dans la tige d'accès (2), la tige d'accès (2) présentant une partie poignée (4) à son extrémité proximale et l'endoscope (8) étant maintenu dans un raccord (6), **caractérisé en ce que** la partie poignée (4) et le raccord (6) sont emboîtés de manière amovible en direction axiale, et **en ce que** le raccord, lorsqu'il est relié à la partie poignée, peut tourner par rapport à la partie poignée (4) autour de l'axe longitudinal de l'endoscope (8) et se déplacer axialement.

2. Système endoscope et tige selon la revendication 1, dans lequel la partie poignée (4) et/ou le raccord (6) sont conçus de façon telle que l'axe longitudinal de l'endoscope (8) maintenu dans le raccord (6) est décalé parallèlement par rapport à l'axe longitudinal de la tige d'accès (2).

3. Système endoscope et tige selon l'une des revendications précédentes, dans lequel la tige d'accès (2) a une forme de section transversale qui présente, dans une première direction, une plus grande largeur que dans une deuxième direction perpendiculaire à la première direction.

4. Système endoscope et tige selon l'une des revendications précédentes, dans lequel la tige d'accès (2) est reliée de manière amovible à la partie poignée (4).

5. Système endoscope et tige selon l'une des revendications précédentes, dans lequel la partie poignée (4) présente, à son extrémité distale, un logement (14) pour recevoir l'extrémité proximale de la tige d'accès (2) et, à son extrémité proximale, un logement (20) pour recevoir le raccord (6), ces deux logements (14, 20) étant disposés de façon décalée l'un par rapport à l'autre dans une direction (e) transversale à l'axe longitudinal de la tige d'accès (2).

6. Système endoscope et tige selon l'une des revendications précédentes, dans lequel la partie poignée (4) présente, à l'extrémité proximale, un manchon (20) qui, à partir de l'extrémité distale, s'engage à l'intérieur du raccord (6).

7. Système endoscope et tige selon l'une des revendications précédentes, dans lequel le raccord (6) présente, à son extrémité proximale, un logement de fixation (38) pour la fixation amovible de l'endoscope (8).

8. Système endoscope et tige selon la revendication 7, dans lequel l'endoscope (8) est fixé de manière amovible sur au moins une surface d'appui (40) du logement de fixation (38) par une bague de serrage rotative (44) dont le diamètre intérieur varie sur sa périphérie.

9. Système endoscope et tige selon la revendication 8, dans lequel le logement de fixation est formé d'un manchon (26) qui présente une découpe (38) en forme de secteur à l'extrémité proximale, les surfaces frontales du manchon (26) orientées vers la découpe (38) formant des surfaces d'appui (40) de l'endoscope (8).

10. Système endoscope et tige selon l'une des revendications précédentes, dans lequel le raccord (6) présente un dispositif de réglage (30, 32) au moyen duquel l'endoscope (8) peut être déplacé axialement par rapport à la partie poignée (4).

11. Système endoscope et tige selon la revendication 10, dans lequel le raccord (6) présente deux manchons (26, 28) s'insérant l'un dans l'autre et venant en prise mutuellement par l'intermédiaire d'un filetage (30, 32), une bague de réglage (36) étant disposée sur un premier manchon (28) pour rotation et par conséquent déplacement axial du premier manchon (28) par rapport au deuxième manchon (26).

12. Système endoscope et tige selon la revendication 11, dans lequel est disposé entre les deux manchons (26, 28) un élément ressort (48) qui crée une force de serrage entre les deux manchons (26, 28).

13. Système endoscope et tige selon l'une des revendications précédentes, dans lequel la partie poignée (4) et le raccord (6) présentent à l'intérieur un lumen libre (56) s'étendant axialement, lequel est aligné sur le lumen libre (22) à l'intérieur de la tige d'accès (2).

14. Système endoscope et tige selon l'une des revendications précédentes, dans lequel l'endoscope (8) présente, dans la région du raccord (6), une section transversale de forme triangulaire de sorte qu'il remplit une section intérieure du raccord (6) sensiblement uniquement dans un secteur inférieur à 180 degrés.
